# EUROPEAN PATENT SPECIFICATION

(11) **EP 3 685 401 B1**
(45) Date of publication and mention of the grant of the patent: **03.01.2024**
(21) Application number: 18778882.3
(22) Date of filing: 24.09.2018
(51) Int. Cl.: G16H 50/20

(54) **METHOD AND APPARATUS FOR DERIVING A SET OF TRAINING DATA**
VERFAHREN UND VORRICHTUNG ZUM ABLEITEN EINES SATZES VON TRAININGSDATEN
PROCÉDÉ ET APPAREIL DE DÉRIVATION D'UN ENSEMBLE DE DONNÉES D'APPRENTISSAGE

(30) Priority: 22.09.2017 GB 201715336
(43) Date of publication of application: 29.07.2020
(73) Proprietor: Optellum Limited, Oxford, Oxfordshire OX1 1BY (GB)
(72) Inventor: KADIR, Timor, Oxford OX2 9JB (GB); PICKUP, Lyndsey, Oxford, Oxfordshire OX2 OED (GB); POTESIL, Vaclav, Oxford OX4 1ER (GB); DECLERCK, Jerome, Oxford, Oxfordshire OX2 7NU (GB)
(74) Representative: Optimus Patents Limited
(86) International application number: PCT/EP2018/075825
(87) International publication number: WO 2019/057975

(56) References cited:
- WO-A1-2017/092615
- US-B1- 9 536 054

## Description

### Field of the invention

The field of this invention relates to computer-aided systems that are used to support clinicians in healthcare. In particular, it relates to computer-aided Diagnosis (CAD) training system that trains a CAD device to produce an output that indicates a probability of a patient having a medical condition of a malignant pulmonary nodule. The output assists the reading and reporting of medical images by radiologists. The data outputs of such systems may be provided to clinicians, such as a 'referring physician' in a hospital setting.

### Background of the invention

In the field of medical imaging, a variety of technologies can be used to investigate biological processes and anatomy. The following examples are types of scan that may be used to provide medical images: X-Ray; Computed Tomography (CT); Ultrasound (US); Magnetic Resonance Imaging (MRI); Single Photon Emission Tomography (SPECT); and Positron Emission Tomography (PET). Each type of scan is referred to as an 'imaging modality'.

Typically, a scan provides a 'dataset'. The dataset comprises digital information about the value of a variable at each of many spatial locations in either a two-dimensional or (more typically) a three-dimensional space. As a specific example, a CT scan may provide images of the chest of a patient. Such a CT scan might, as a more specific example, show lung nodules in the chest.

Computer Aided Detection (CADe) systems are known and aim to assist clinicians. CADe systems aim to provide a clinician with standardised, objective and repeatable information. That information might typically relate to structures, both normal and lesions, in a person. Examples of CADe systems include the mammography CADe systems available commercially such as *'PowerLook Advanced Mammography Platform'*from 'iCAD' and the 'ImageChecker' from 'Hologic'. The purpose of such products is to feed the most accurate information possible to a radiologist. These products attempt to achieve this by detecting intelligently, in an automated manner, suspicious regions in the image and showing these to the radiologist. The skilled work of the radiologist may therefore be directed to such regions, where his/her experience and judgement can then be brought into play.

CADe systems may be used as a so-called 'Second Reader' system. This phrase is based on an approach whereby a radiologist first looks at an image resulting from a scan, for example a mammogram. The radiologist will then, based on training and experience, identify areas of the scan where the radiologist considers that there may need for further investigation of a medical condition, for example via a biopsy. However, the radiologist can then consider the CADe findings. Those findings might involve a display to highlight any additional suspicious regions on the mammogram. The radiologist will then, based on training and experience, look at those further areas of the scan. The CADe system is thereby, figuratively speaking, performing a 'second look' at the scan. The results of that second look at the scan may be that the radiologist will be directed to areas of the scan that he/she may have overlooked or viewed in a cursory manner. In this way, CADe systems are designed to reduce 'false negatives', which are also termed 'missed findings'. Thus CADe systems perform a valuable support role to clinicians.

Computer Aided Diagnosis (CADx) systems is a related technology to CADe. CADx systems attempt to solve a different problem, and relate generally to risk assessment. Instead of focussing on potentially missed findings, as in CADe, they try to assist the user to classify findings correctly, either as malignant or benign in the case of cancer. They rely on the user, such as a radiologist, to detect, that is identify, abnormalities in a scan but then typically provide a score that is indicative of risk of malignancy. There are many examples of such CADx systems proposed within the academic literature. However, few systems are available commercially, and hence used in clinical practice. This discrepancy is indicative of the difficulties in deploying practical, accurate and reliable systems with the known approaches.

The output of known CADx systems is typically some kind of score. That score indicates a risk or likelihood of disease, or its absence. An example of a commercial CADx system is the *'Transpara'* product from *'Screenpoint'.* There are many non-clinical CADx systems in the academic literature, which includes the references [1] and [2] at the end of this Background section.

In order to characterise the performance of CADx systems, and indeed other medical tests, one is typically interested in their 'sensitivity' and 'specificity'. Sensitivity is the fraction of cases that have the disease that the CADx system correctly identifies, whereas specificity is the fraction of cases that do not have the disease that the CADx system correctly identifies. These two terms are also called 'True Positive Rate' and 'True Negative Rate' and an ideal CADx system should have 100% Sensitivity and 100% Specificity, which is typically an unachievable goal. In practice, a CADx system's performance can usually be adjusted to trade sensitivity against specificity by adjusting the so-called 'Operating Point', which can be a threshold on the output of the system. In more detail, a threshold may be set on the CADx output score and then when, for a particular patient, the score is greater than this threshold, it can be interpreted as indicating that the patient has the condition. Conversely, if the score is below this threshold, it can be interpreted as indicating that the patient does not have the disease. In this manner, the threshold specifies the condition/no-condition decision point of the CADx system, referred to as the 'Operating Point', and controls the balance between sensitivity and specificity of the CADx system.

However, this known prior art has a number of limitations. The most notable limitation is that CADx systems are invariably designed to optimise the average number of correct diagnosis classifications made on the datasets used for training and testing. This is because the training algorithms typically consider each element of the training set equally when evaluating the performance of the classifier during training. However, in some clinical applications, clinical users are more concerned with other factors, such as the optimal use of resources or health economic cost-effectiveness of clinical decisions. Such considerations may not correspond to the maximisation of the average classification performance and, therefore, the prior art training methods will result in CADx systems that are sub-optimal in practice for certain applications. For example, it may be better that the CADx system maximises the specificity under the constraint that the sensitivity is 100%. An example of one such application is identified with a case of managing patients that present with one or more Pulmonary Nodules (PNs) on Chest Computed Tomography (CT). A PN is a small mass that is visible in the CT image, typically 5mm to 30mm in diameter. They are relatively common findings in Chest CT imaging, with a typical incidence between 20-30% of all Chest CTs, but where the vast majority, greater than 98%, are benign. The remaining incidences are cancerous. Numerous physiological processes result in benign PNs; some arise from disease-related events, such as bacterial or viral infections, whereas others are normal physiological processes, such as enlarged lymph-nodes.

PNs are typically detected in one of three situations: 1) in lung cancer screening, as pioneered in the US National Lung Screening Trial; 2) as incidental findings on Chest CTs performed for non-malignant disease; or 3) in monitoring of cancer survivors.

Regardless of the patient pathway to presentation, once detected, the reading radiologist must stratify the PN into one of a number of broad categories ranging from highly suspicious of malignancy through to definitely benign, and make a recommendation for the next step in the management of the patient. The recommendation depends on the clinical context and pathway of the patient, screening, incidental or cancer survivor. However, the recommendations typically fall into one of three broad categories:
(i) No follow-up imaging needed: for cases where the radiologist is confident that the nodule is definitely benign;
(ii) Short-term, e.g. 3 months, or long-term, e.g. 9 months, follow-up CT imaging is recommended to assess any change in size, volume or appearance: for cases where the radiologist is not confident that the PN is definitely benign. These are often termed 'indeterminate nodules'; and
(iii) Additional investigation using imaging or tissue sampling is recommended 'now', for cases where the radiologist feels that the PN has characteristics that are suspicious of cancer.

Radiologists often struggle to determine whether a PN is malignant or benign from a single Chest CT scan and therefore, the majority of PNs will be deemed 'indeterminate' and recommended for follow-up. Given that the vast majority are in fact benign, this leads to many unnecessary and time-consuming follow-up scans, additional radiation exposure and stress to the patient. Thus, many such indeterminate nodules are referred for up to 5 follow-up CT scans, to assess changes over time, before they can be ruled as benign. In one review of the management of incidental PNs, out of 55,435 patients that had a PN that were detected and monitored, only 6.2% eventually had a diagnosis of lung cancer, as published in "Recent Trends in the Identification of Incidental Pulmonary Nodules", authored by Michael K. Gould, Tania Tang, In-Lu Amy Liu, Janet Lee, Chengyi Zheng, Kim N. Danforth, Anne E. Kosco, Jamie L. Di Fiore, and David E. Suh, published in American Journal of Respiratory and Critical Care Medicine Volume 192 Number 10 | November 15 2015.

There are national and international guidelines to assist the radiologist in making such decisions, including: (i) The British Thoracic Society, "BTS Guidelines for the Investigation and Management of Pulmonary Nodules", In Thorax. August 2015 Volume 80 Supplement 2; (ii) Guidelines for Management of Incidental Pulmonary Nodules Detected on CT Images: From the Fleischner Society 2017. Heber MacMahon et al. Radiology, found at: http://dx.doi.org/10.1148/radioI.2017161659; and (iii) Lung CT Screening Reporting and Data System (Lung-RADS™) found at: https://www.acr.org/Quality-Safety/Resources/LungRADS American College of Radiology.

Clinical centres may choose to adopt one of the above recognised guidelines, adapt them to their local needs or develop their own. However, even following such guidelines, radiologists typically recommend many follow-up imaging tests for, what ultimately prove to be, benign nodules. For example, the updated Fleischner guidelines recommend that every solitary solid PN between 7-8mm is followed at 7-12 months and then again at 12-24 months. Those PN values that are greater than 8mm should be followed up at 3 months and/or CT-PET, and/or biopsy performed.

In addition to the institutional guidelines and practices of the centre, the experience or specialism of the reading radiologist plays a significant factor in the fraction of PNs that are recommended for follow-up. For example, a thoracic radiologist with 10 years' experience reading only Chest CTs will be able to confidently determine benign perifissural lymph-nodes - normal lymph-nodes that appear near the fissures between the lobes. However, almost by definition, many incidental findings are detected by radiologists that are specialists in other diseases, such as cardiac disease or pulmonary embolism. Less experienced radiologists have also been identified as being more cautious in their recommendations.

Hence, the inventors have recognised and appreciated that a CADx system could be created and/or trained to better assist the non-specialist or less experienced radiologist in confidently and consistently making benign determinations.

However, the inventors have also recognised and appreciated that for most effective decision making, the CADx system must be trained and optimised to assist the radiologist to reduce the fraction of PNs that are deemed to be indeterminate. More specifically, the CADx system should be optimised to maximise the True Negative fraction, the number of true benigns that are correctly classified as benign, whilst having a 100%, or near 100%, sensitivity (True Positive fraction) i.e. not mis-classifying any true malignants as benign.

The problem is exacerbated when the CADx system is to be updated with an improved performance. Since many such systems are built using machine learning technology, any improvement in the performance of the system, or even a significant change in the way in which scores are produced without an improvement in performance, will require that the clinician re-adapts their decision making. For example, a change to the training data will result in different scores for the same input image. The conventional solution to adopt such system updates/improvements is to provide the user with a threshold that they can apply to the score; for instance, scores above this threshold should be considered indicative of disease and those below should not. This approach is analogous to that used for other medical tests, such as measuring the level of cholesterol in the blood as an indicator of heart disease risk where certain thresholds are used to stratify patients into low, medium and high risk. Similarly, numerous schemes have been devised to standardise the reading and reporting of findings in Breast (BIRADS) and Lung imaging (LungRADS) that use thresholds agreed by clinical experts to stratify patient risk. However, while such a solution is conventional in the field, it still has the disadvantage that the clinicians in the medical field need to understand and agree the thresholds used. This can take a long time and presents a significant barrier to building trust in the system and to gain adoption.
[1] The document by Sun T, Zhang R, Wang J, Li X, Guo X (2013) titled 'Computer-Aided Diagnosis for Early-Stage Lung Cancer Based on Longitudinal and Balanced Data. PLoS ONE 8(5): e63559. doi:10.1371/journal.pone.0063559 describes a system that is designed to produce an output between -1 and 1. For example, see the section 'Methods, sub-section Prediction Model 'where *Yᵢ* ∈ {+1, -1} is the corresponding desired output'.
[2] The document by Way T, Chan H-P, Hadjiiski L, et al. titled 'Computer-Aided Diagnosis of Lung Nodules on CT Scans: ROC Study of Its Effect on Radiologists' Performance', published in Academic radiology, 2010; 17(3):323-332. doi:10.1016/j.acra.2009.10.016 describes a system produces an output between '1' and '10', as described in the section Methods and Materials, the last paragraph in CAD System, 'The original bin value was mapped linearly as a relative malignancy rating on a scale of '1' to `10".

As discussed earlier, a conventional CADx system built using prior art machine learning training methods is designed to optimise average performance across all examples in the training set. Therefore, such prior art methods cannot be used to maximise the True Negative fraction, the number of true benigns that are correctly classified as benign, whilst having a 100%, or near 100%, True Positive fraction i.e. not misclassifying any true malignants as benign. US 9,536, 054 B1 describes a CAD training system using training images with malignant lesions. This citation describes a computer aided diagnosis training system, for training a CAD device, where the CAD device comprises an input circuit configured to receive and assemble training data that comprises: medical data of patients that have been identified as having at least one medical condition, and medical data of patients that have been identified as not having the at least one medical. In the method of this citation a confidence level indication is provided to minimize deviations in recommended clinical actions due to biases of the group or individual in interpreting the rules of the system. WO2017/092615 describes a computer aided diagnosis system that uses a classifier training method where the classification targets in the training images are segmented in order to extract features, and a subset of the features are used to train the target classifier.

Thus, the inventors have recognised and appreciated that there exists a desire for an improved Computer Aided Diagnosis (CADx) system to assist the reading and reporting of medical images by radiologists and the interpretation of the radiologist's report by the physician responsible for patient care - the so-called 'referring physician'. More specifically, examples of the invention relate to the management of Pulmonary Nodules (PNs) detected on Chest Computed Tomography (CT) scans by radiologists.

### Summary of the invention

Accordingly, the invention seeks to mitigate, alleviate or eliminate one or more of the abovementioned disadvantages singly or in any combination. The invention is set out by the appended set of claims.

According to a first aspect of the present invention, there is provided a Computer Aided Diagnosis, CAD, training system for training a CAD device to produce an output that indicates a probability of a patient having a medical condition of a malignant pulmonary nodule as claimed in claim 1. In this manner, a parsing circuit and data classifier circuit are configured to train the CAD device to distinguish between input medical images based on clinical data and/or attributes of the input medical images to better produce an output from the CAD device that indicates a probability of a medical condition of a patient. The data classifier circuit produces an output from the CAD device that indicates a probability of a medical condition of a patient based on the prioritised subsets that share the at least one clinical attribute or radiological attribute.

In some optional examples, the classifier circuit may include a feature extraction circuit arranged to extract features of the separated assembled training data and use data analysis techniques to identify features of patient medical data that group together as being easier to classify in relation to the at least one medical condition that can be discarded from further review by a clinician. In some optional examples, the identified features of patient medical data that group together comprise features that are a distance greater than a feature space threshold from positive cases that require further review by a clinician. In some optional examples, the data classifier circuit may be configured to update weights associated to the separated assembled training data over iterative training operations. In some optional examples, the medical data may include at least one from a group of: medical images, blood test results, genetic profiling, relevant to the diagnosis of pulmonary nodules.

According to a second aspect of the invention, there is provided a method of training a Computer Aided Diagnosis, CAD, device as claimed in claim 6 .

### Brief description of the drawings

Further details, aspects and embodiments of the invention will be described, by way of example only, with reference to the drawings. In the drawings, like reference numbers are used to identify like or functionally similar elements. Elements in the figures are illustrated for simplicity and clarity and have not necessarily been drawn to scale.
FIG. 1 illustrates a simplified block diagram of an example set of all datasets that may be classified within a training set of an example computer-aided risk assessment system in a medical application, according to examples of the present invention.
FIG. 2 illustrates a simplified flowchart of an example of a method for deriving a set of training data in a medical application, according to examples of the present invention.
FIG. 3 illustrates an example of a CADx device based on Support Vector Classification, according to examples of the present invention.
FIG. 4 illustrates an example of a Computer Aided Diagnosis device training system, according to examples of the present invention.
FIG. 5 illustrates an example of using radiological classifications to identify the EN labels, according to examples of the present invention.
FIG. 6 illustrates an alternative example of a simplified architecture that uses automatic detection, segmentation feature extraction followed by a clustering algorithm in order to identify groups of nodules that have similar characteristics, according to examples of the present invention.
FIG. 7 illustrates an example of the output of a clustering algorithm visualised in two dimensions, according to examples of the present invention.
FIG. 8 illustrates an example of a determination of easier and difficult negative cases in a two dimensional feature space, according to examples of the present invention.

### Detailed description

Examples of the invention propose a computer-aided diagnosis (CAD) training system and method to train a CAD device to provide a better output of data based on a range of medical mage data and clinical data.. In particular, the invention proposes a parsing circuit configured to separate the assembled training data into data sets, such that a first data set contains only the medical data of patients that have the at least one medical condition and a second data set contains only the medical data of patients that do not have the condition. Thereafter, the invention parses at least one of the data sets into at least two subsets, whereby a first subset is distinguished over a second subset of the at least two subsets by at least one attribute, for example whether the medical image contains benign attributes or malignant attributes. The invention further includes a data classifier circuit configured to associate different weights to the separated assembled training data, such that the first subset is prioritised during training of the CAD device. Such weighting is applied to train the CAD device, such that the CAD device is trained to more correctly classify medical images. In this manner, a parsing circuit and data classifier circuit are configured to train the CAD device to distinguish between input medical images based on clinical data and/or attributes of the input medical images to better produce an output from the CAD device that indicates a probability of a medical condition of a patient.

In contrast to known techniques, some examples of the invention further describe identifying the subsets of training data using expert opinion to provide a trained CADx system that consistently assesses subsets of cases. Here, an expert means a radiologist that has many years' experience in interpreting and reporting medical images of the speciality of interest. For example, in the application of the management of pulmonary nodules and lung cancer, this would be a thoracic radiologist specialising in lung cancer.

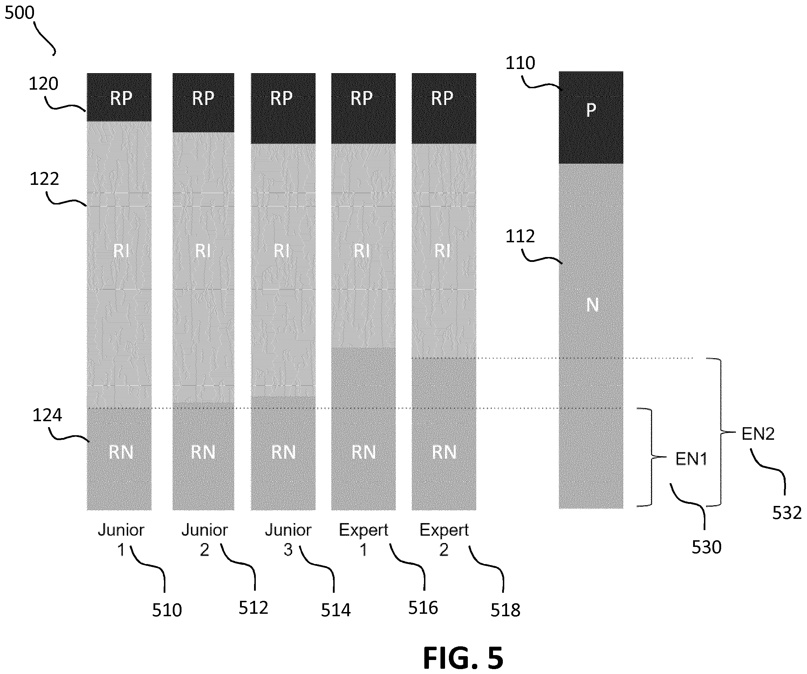

Examples of the invention also propose a CAD training system and method to train a CAD device, in order to identify the above subset by measuring expert performance in classifying cases. Expert performance may be measured by asking radiologists or other suitably qualified clinicians to review a set of cases, where the true diagnosis is known and to state whether they think each case is Radiologically Positive (RP) meaning that they consider the patient to have the condition based on the image, Radiologically Negative (RN), meaning that they consider the patient not to have the condition based on the image, or Radiological Indeterminate (RI) meaning they are not sure whether the patient has or does not have the condition. In general, the experts will have differing performance as illustrated in FIG. 5, and this may be utilized to identify data subsets as is described below.

Examples of the invention further propose a CAD training system and method to train a CAD device to identify the above subset by examining the feature space within which the training system represents the training data. Examples of the invention further propose a CAD training system and method to train a CAD device using an iterative approach to identify the above subset by measuring the CAD training system performance trained in a first run of receiving and assembling training data that includes medical data of patients that have been identified as having at least one medical condition, and medical data of patients that have been identified as not having the at least one medical condition. Once a first performance of the CAD training system has been determined and resulted in improved parameters, the CAD training system is re-run to further improve the results with a new set of training data, and so on.

In some examples, a CADx system may be used to output data that assesses the risk of a patient having a condition from medical data images. Hence, references to a CAD training system or computer-aided risk assessment system hereafter encompasses a CADx system or a risk model that is configured to identify a risk of a patient having a medical condition.

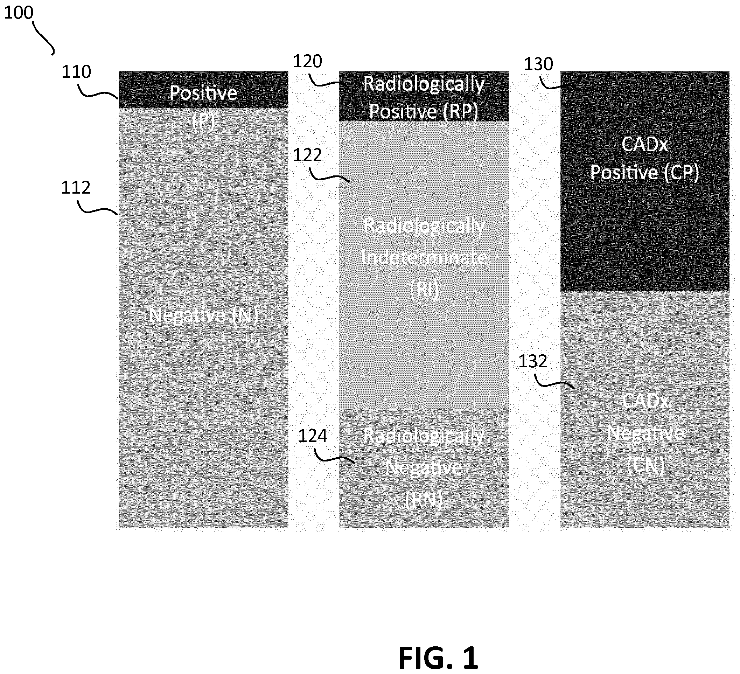

Referring now to FIG. 1, an example set 100 of all datasets that may be classified within the training set of an example CAD training system is illustrated, according tothe invention. Here, negative cases (N) 112 are defined as the set of cases within all cases (AC) in which the disease is absent; and positives cases (P) 110, are defined as the set of cases within AC in which the disease is present. Further cases may be defined as: Radiologically Positive cases (RP) 120, identifying the set of cases that are considered by the radiologist to definitely contain disease; Radiologically Negative cases (RN) 124, identifying the set of cases that are considered by the radiologist to definitely not contain disease; and Radiologically Indeterminate cases (RI) 122, identifying the set of cases that are considered by the radiologist as neither definitely positive nor definitely negative, but are recommended for follow-up imaging. In response to the improved training approach of the CAD training system, as described herein, the CAD training system will output a more accurate proportion of CAD Positive cases (CP) 130, identifying the set of cases that are considered positive by the CADx system; and CAD Negative cases (CN) 132, identifying the set of cases that are considered negative by the CADx system.

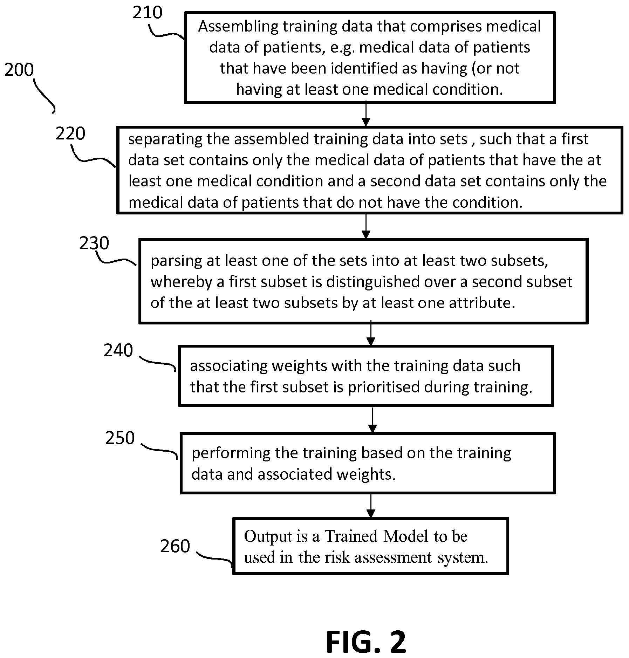

Referring now to FIG. 2, a flow chart illustrates a method of training a CAD device in a CAD training system, according to some examples of the invention. The method 200 for training a CAD device includes assembling training data that comprises medical data of patients, and in particular medical data of patients that have been identified as having at least one medical condition, and medical data of patients that have been identified as not having the at least one medical condition, at 210. The method 200 for training a CAD device further includes separating the assembled training data into sets, such that a first data set contains only the medical data of patients that have the at least one medical condition and a second data set contains only the medical data of patients that do not have the condition at 220. The method 200 for training a CAD device further includes parsing at least one of the data sets into at least two subsets, whereby a first subset is distinguished over a second subset of the at least two subsets by at least one attribute at 230. The method 200 for training a CAD device further includes associating different weights, at 240, to the separated assembled training data, such that the first subset is prioritised during training. The method 200 for training a CAD device further includes performing the training based on the training data and associated weights at 250. The result or output of the training operation is a trained model that is used in computer-aided risk assessments, as shown at 260.

In some examples, the separated subsets of training data may be configured to contain only patients with the same attribute, which may include benign and malignant attributes, radiological appearance, patients with the same type of disease versus different diseases, patients with the same risk categories of disease versus different risk categories where a risk category is a range of risk values, patients with the same history versus those with different histories, where one example of a history may be their smoking history or history of prior disease. The term 'attributes' used herein is therefore intended to at least encompass one or a combination of any of the above examples, as well as equivalents or similar medical related conditions.

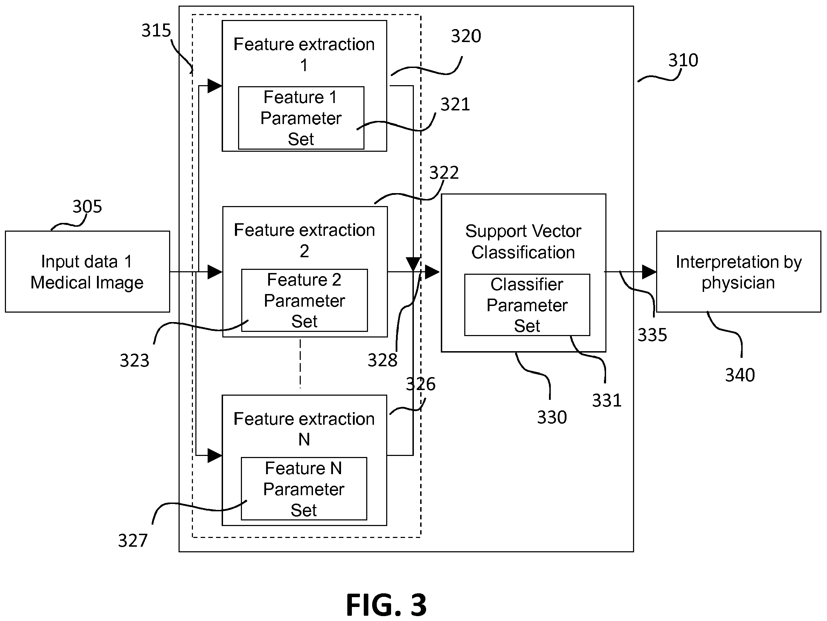

Referring now to FIG. 3, an example of a CADx device 310 based on a Support Vector Classification circuit 330 and based on machine learning is illustrated, according to examples of the present invention. In some examples, the functionality provided by the Support Vector Classification circuit 330 may be implemented as an algorithm, or in firmware or hardware, such as a processor configured with (or adapted to run) a particular program to associate different weights to separated, assembled training data, such that a first subset is prioritised during training of the CADx device 310. Aspects of the invention employ machine learning techniques, but notably modify the training of the classification or regression systems that use machine learning.

In this example, the CAD device includes an input circuit 305 that contains at least one medical image and clinical data, and the CADx device 310 produces an output 335 that is an indicator, e.g. a data flag, showing a result of a classification, e.g. '0' for benign and '1' for malignant, or more generally a score, the value of which is related to a probability that the patient has the condition that the CADx device is trained to analyse. In this manner, the output 335 of the CAD device training system 310 is provided to clinical physicians 340, such as radiologists, in order to assist the reading and reporting of medical images and the interpretation of the radiologist's report by the physician responsible for patient care - the so-called 'referring physician'. More specifically, examples of the invention may relate to the management and classification of Pulmonary Nodules (PNs) detected on Chest Computed Tomography (CT) scans by radiologists.

Alternatively, in some examples, a regression algorithm may be used instead of the classifier circuit 330 in cases where the output needs to be more complex than a binary indicator, for example a real number indicating the risk of disease. Thus, it is envisaged that some examples of the invention may work with either classification or regression based CADx systems, dependent upon the data being processed and/or the application. Such classification and regression CADx systems, as envisaged herein, require a training step prior to their use. Training involves a use of an automatic training algorithm employed by classifier circuit 330 and a corpus of training data, for example medical data of patients that have been identified as having at least one medical condition, and medical data of patients that have been identified as not having the at least one medical condition, and therefore where the desired output is known. The classifier circuit 330 adjusts the parameters of the classification or regression algorithm so as to minimise the misclassification error, sometimes called a loss function, which is typically based on the fraction of training example cases that have been incorrectly classified to the total number of training examples.

One drawback of the known approach is that it treats all examples in the training set equally, and hence the resulting classifier becomes optimal on average, e.g. it evolves to classify an overall average for the data content, rather than specific attributes of the data.

However, the inventors of the present invention have recognised and appreciated that, in practice, it may be that for a particular application there is interest in correctly classifying certain examples in the training set over others. In some examples, therefore, it is envisaged that it may be important to achieve a very accurate classification of the malignant cases, or some particular subset of them, whereas misclassification of the benigns is acceptable.

To achieve this, a known method is to use weights on the loss function so that certain training examples contribute by a greater degree to the loss function than others. Known as a weighted loss function, this approach can increase the penalty for misclassification of one class over the other (or others). This approach can also account for unbalanced data, where there are many more of one class, e.g. benign medical data scans of a medical conditions, than the other class. However, this known approach cannot be used to directly solve the problem described above because it is always applied to the whole class, and not to subsets of data within a class, as described in examples of the invention. In an embodiment the invention,, e.g. the easier benign examples are identified, and these are selected to be weighted more than the other benigns. However, it is not known which should be weighted to a greater or less degree in advance. Thus, some examples of the invention further provide a CAD training system and method for a CAD device to solve this by estimating the classification difficulty of each element in the training set.

Some examples of the invention may also solve the problem of maximising the number of negative cases that are correctly classified whilst ensuring that 100% (or nearly-100%) of positive cases are correctly classified. The CADx device 300 does this by identifying a subset of negative cases that are in some way easier for the classifier circuit 330 to correctly classify than others in the negative set, and then weighting these to a greater degree than the others. In this manner, the examples herein described differ from the prior art teaching of simply weighting one class, e.g. malignant, greater than the other, e.g. benign, as the illustrated examples are able to divide each of the classes into multiple subsets, each of which may be configured to have their own weights. Some examples of the invention refer to this subset as Easier Negative (EN).

Note that by swapping the positive and negative labels it is trivial to optimise the opposite problem, e.g. classifying malignant (EP) instead of benign (EN), should the application require this. However, for simplicity purposes only, the description herein focuses on the former, whilst it is envisaged that the concepts described herein also encompass the latter.

The classifier circuit 330 is configured to parse at least one of the data sets of separated, assembled training data into at least two subsets 328, whereby a first subset is distinguished over a second subset of the at least two subsets 328 by at least one attribute. Examples of the invention disclose a number of different ways in which the subset EN can be identified:
1) A first approach is to use expert knowledge of clinicians to identify a subset of negative cases with distinct and consistent traits;
2) A second approach is to use a panel of radiologists with different levels of expertise, specialism and experience to classify all the cases (AC) into RP, RI and RN. The differences between the consensus classifications of highly experienced and specialised radiologists and those more junior can be used to define EN;
3) A third approach is to identify EN by examining the databases within the clinical setting, e.g. the Picture Archival and Communication System (PACS), Radiological Information System (RIS) and Electronic Medical Record (EMR). In some examples, negatives with similar disease characteristics can be matched and then the CADx device 300 can track what happened to these patients and which radiologists read the scans. Statistical differences between expert and junior readers for similar negatives may be used to identify those that should be EN;
4) A fourth approach uses the features extracted by the classifier circuit 330 to map the cases in AC into a feature space. In this approach, the classifier circuit 330 may then use data analysis techniques, such as dimensionality reduction, embeddings and cluster analysis, to identify those features that group together and are likely to be easier for the classifier circuit 330 to learn, and hence define EN; and
5) A fifth approach is to iteratively train the classifier circuit 330 and to identify EN during the training process. For example, this approach may work, in some examples, by selecting a subset of negatives that are misclassified, but where they are sufficiently far away in feature space from the positive cases, and hence are less likely to be misclassified.

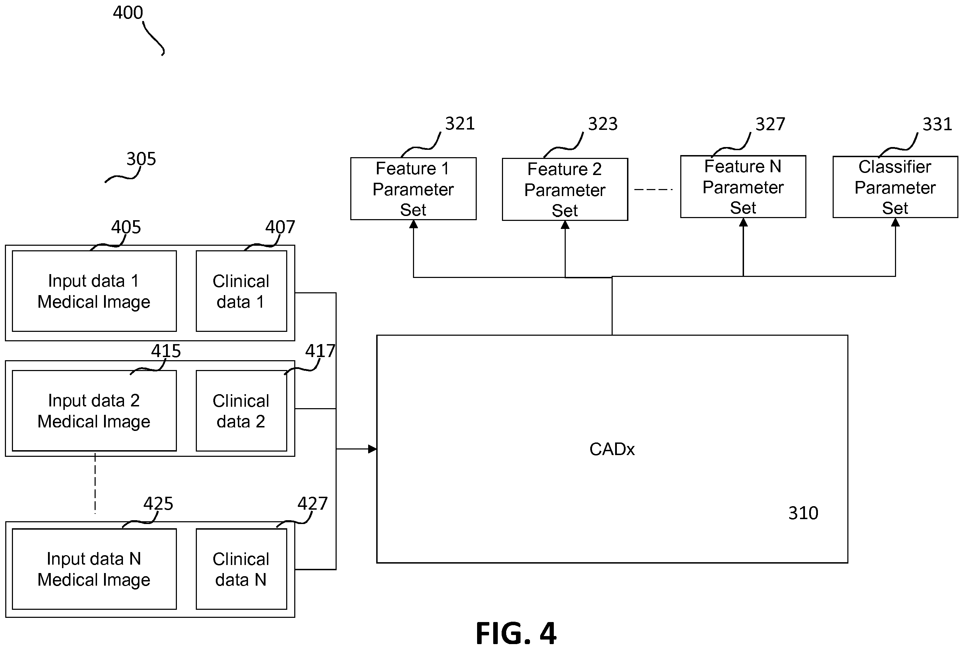

Referring now to FIG. 4, a block diagram example 400 of a Computer Aided Diagnosis, CAD, device training system 400 is illustrated, according to examples of the present invention. The CAD device training system 400 includes a CADx system 310 that comprises (or is operably coupled to, as shown) a number of input circuits configured to receive and assemble training data that comprises medical data of patients and attributes related to the patients' medical condition. In this example, the received and assemble training data includes a first medical image 405 and medical data of at least one first patient and at least one attribute related to the at least one first patient's medical condition 407, a second medical image 415 and medical data of at least one second patient and at least one attribute related to the at least one second patient's medical condition 417 and so on up to an N^{th} medical image 425 and medical data of an N^{th} patient and at least one attribute related to the N^{th} patient's medical condition 427.

In FIG. 4, the CADx system 310 includes a parsing circuit, such as parsing circuit 315 of FIG. 3, coupled to the input circuit and configured to separate the training data into sets, such that each set contains only patients with the same attribute related to the patients' medical condition. The parsing circuit (not shown) is further configured to parse at least one of the sets into at least two subsets 328, where a first subset is distinguished over a second subset of the at least two subsets 328 by at least one of a clinical attribute or a radiological attribute, in relation to the at least one medical condition as patient medical data that can be discarded from further review by a clinician.

In FIG. 4, the CADx system 310 includes a data classifier circuit, such as data classifier circuit 330 of FIG. 3, coupled to the parsing circuit and configured to associate weights with the assembled and separated training data such that subsets that share the at least one attribute are prioritised during a training process as being easier to classify as data in relation to the at least one medical condition as patient medical data that can be discarded from further review by a clinician.

In some examples, as described in the fifth approach above, the CAD training system 310 and method to train a CAD device may use an iterative approach to identify the above subset by measuring the CAD training system performance trained in a first training run of receiving and assembling training data that includes medical data of patients that have been identified as having at least one medical condition, and medical data of patients that have been identified as not having the at least one medical condition. Once a first performance of the CAD training system has been determined, and resulted in improved parameters, the CAD training system is re-run to further improve the results with a new set of training data, and so on.

The CADx system 310 outputs various feature parameter sets 321, 323, 327 to a Support Vector Classification circuit (not shown).

In this manner, the various feature parameter sets 321, 323, 327 are iteratively optimized according to the classifications identified in FIG. 5. In particular, the CADx system 310 adjusts the associate weights applied to the separated assembled training data such that the Computer Aided Diagnosis device training system 400 focuses on a classification of some example attributes of medical images over others.

### Example 1

In this example t, expert knowledge is used to identify groups within 'N' that share some clinical or radiological characteristics, and hence are likely to be easier for a classifier, such as classifier circuit 330, e.g. a Support Vector Classifier, to learn. In FIG. 3, the CADx device 300 receives and assembles training data that comprises medical data of patients and clinical data, such as attributes related to the patients' medical condition.

The CADx device 300 implements a separation of training of medical data, of patients and attributes related to their medical condition, into sets such that each set contains only patients with the same attribute in feature extraction circuits 320, 322, 326. In some alternative examples, this operation may be provided by one or more processors (not shown) located prior to feature extraction circuits 320, 322, 326. Similarly, in FIG. 3, the CADx device 300 implements the parsing of at least one of the sets into at least two subsets 328, where a first subset is estimated to be easier to classify than a second subset. In feature extraction circuits 320, 322, 326. One or more attributes distinguish between the first subset and second subset is/are a clinical attribute or a radiological attribute and subsets that share the at least one attribute are prioritised during a training process as being easier to classify as data in relation to the at least one medical condition as patient medical data that can be discarded from further review by a clinician.

In the example of FIG. 3, each feature extraction circuit 320, 322, 326 includes its own respective parameter set 321, 323, 327 that specifies the operation feature extraction circuit 320, 322, 326. Therefore, in some examples, feature extraction circuits 320, 322, 326 may be implemented using filter circuits, such as a finite impulse response (FIR) digital filter, for example whereby the filters may be defined as a series of values for a convolution operation. In this example, the Support Vector Classifier 330 may also include an internal classifier parameter set 331, which define the Support Vector Classifier's 330 behaviour. It is envisaged that the CADx device 300 may include some thousands or tens of thousands of such parameters that are to be set. It is envisaged that in some examples, in very modern systems that use Convolutional Neural Networks, the CADx device 300 may include millions of such parameters.

Classifier circuits, such as Support Vector Classifier 330, operate best in cases where the classes have consistent characteristics within the class and distinct characteristics to the other class. For example, in the case of PNs, a subset of the benign nodules that appear in Chest CTs are the so-called perifissural lymph nodes, which are completely normal anatomy. They can be identified by their location, near the lung lobe fissures, and their characteristic appearance. They are relatively straightforward for the expert Chest radiologist to distinguish, but difficult to the non-expert or non-Chest CT specialist.

In this embodiment, one or more of such subsets are identified by experts and labelled in Support Vector Classifier 330 as the EN set. In some examples, this may be a binary label, i.e. the case is either considered to be in EN, or not. Alternatively, in some examples, the subset(s) can be implemented as a weight, whereby each case is assigned a number indicating the degree of membership of EN, which is the degree to which the expert considers them easier to classify than others. In this example, one or more experts may perform the labelling or weighting such that the consensus sum or other combination of the expert scores can be used to extract the labels or weights. For example, if all the experts agree that a case belongs to EN then this can be given a weight of 10, whereas if only 1 out of 10 consider it to belong to EN then this can be given a weight of 1.

In examples of the invention, the raw input data from the expert(s) is then processed in order to arrive at a subset EN. As discussed above this may be performed by averaging, using the experts' opinion in a voting scheme or some other aggregating operation. In some examples, the raw input data from the expert(s) may also be processed in a non-linear manner, such that the data from some expert's opinions is prioritised over other expert's raw input data, for example perhaps based on the relative expertise of the expert. In some examples, the raw input data from the expert, even once processed, may not be sufficient to use in isolation in order to define EN, and in this instance it is envisaged that some additional processing of the data, such as thresholding, may be needed in order for the CADx system to select EN.

Once the set of EN has been identified, this is used to adjust the weight of the training cases within EN to train the classifier 330 or regressor. For instance, cases in P are given a weight of '1', those in EN a weight of '1' and the remaining in N, N - EN, a weight of '0.5'. This would encourage the classifier 330 to consider the positive and easier negative to a greater degree than the negatives not in EN.

### Example 2

In this second example a panel of radiologists with different levels of expertise, specialism and experience classify all the cases (AC) into RP 120, Rl 122 and RN 124. The differences between the consensus classifications of highly experienced and specialised radiologists and those more junior personnel can be used to define EN. This implementation is illustrated in FIG. 5, using radiological classifications to identify the EN sets 530, 532. Here, in this example, 6 radiologists formed of 3 juniors 510, 512, 514 and 2 expert 516, 518 levels have categorised each case in AC into one of RP 120, Rl 122 and RN 124. Note that in this illustration, the radiologists' classifications are strict supersets or subsets of each other. In practice, it is envisaged that in some cases the classifications may be classified totally differently by each radiologist.

There are several ways in which the EN set(s) 530, 532 may be defined. In the subset labelled as EN1 530, examples of the computer-aided risk assessment may select the negative cases 112 that all radiologists agree are negative as an output. These are highly likely to be those that the classifier will be able to correctly classify since all radiologists agreed. However, since all radiologists agreed on all such cases, the output of the CADx system is unlikely to add much clinical benefit, except perhaps to those who are newly qualified.

The subset labelled as EN2 532 is defined as those cases for which all experts agreed are definitely negative and includes those cases that all radiologists agreed were negative. The classifier circuit is likely to correctly classify these cases, because all experts agreed on the classification.

The final step is to use the weighted loss function to set up the training of the classifier as with the first embodiment. This second approach has an advantage over the first embodiment in that it attempts to estimate each case's likelihood of correct classification by using empirical human performance rather than expert judgement.

### Example 3

The third example which does not fall under the scope of the claims, further develops the second embodiment by automating the process of identifying the EN subset based on information within the hospital information technology (IT) systems; the Picture Archival and Communication System (PACS), Radiological Information System (RIS), Electronic Medical Record (EMR) and Radiological Reporting System. Some of the key steps in this implementation are:
1) To identify groups of patients whose characteristics, or those of their disease, are similar to others in each group;
2) To identify which radiologists are reading the images for those patients along with their relative experience and specialism; and
3) To track what happens to those patients.

The above information can be used select the subset or subsets of patients who are consistently misclassified by junior radiologists, but that are correctly classified by experts. This approach is illustrated further using the PN use-case as follows
1) Automatically search the RIS and PACS for all cases within a predefined range of dates in the past that have had a nodule reported. The dates need to be such that a two year record of all patients is possible. Practically, this can be done with good reliability by using Natural Language Processing (NLP) to search the appropriate terms in the radiological report, typically held in the RIS. By performing queries on the PACS and RIS, the number of follow-ups and associated reports can be determined.

To determine similar groups of patients, examples of the computer-aided risk assessment system may specify a set of predefined nodule characteristics that can be matched with those that have been reported by the reading radiologist, such as size. Alternatively, the system may automatically retrieve the images from the PACS, perform automatic nodule detection and segmentation to extract features that characterise the size, appearance and shape. The system can then identify groups of nodules through the use of automatic clustering algorithms. An alternative to this is envisaged to include automatically detecting a subset of specific nodule types, e.g. a perifissural nodule using a classifier that has been previously trained on labelled training data.

As a specific example of this step, the system can group nodules of similar size together. As discussed earlier, expert radiologists are able to better categorise benign nodules in the range of 4-14mm than non-specialists or juniors.
2) In some examples, the experience and specialism of each radiologist in the clinical centre can be provided as prior information to the system. The identity of the reading radiologist reading each study is typically recorded in the RIS and EMR and can be readily retrieved.
3) Finally, by tracking and linking the studies in the PACS, RIS and EMR it is possible to establish how many follow-ups a particular patient underwent. By linking this information with that obtained in the above steps 1) and 2), it is possible to establish which types of nodules are most consistently misclassified by junior radiologists, but which are correctly classified by experts. In more detail, those patients who present with similar nodules should have similar follow-up schedules. By examining the differences between the follow-up schedules in those patients and mapping to the experience of the reading radiologists it is possible to automatically identify the subset that can be used to assign to the EN subset.

### Example 4

The fourth example of the invention operates by first extracting features from the images of patients, for example using feature extraction circuits 322, 324, 326 in FIG. 3, then analysing the distribution of said features to identify those that are similar to each other. In this example of a CADx device 300, patients whose disease appearance is similar to one another will be easier for the classifier 330 to learn. In some examples of the invention, these features are derived from regions in the image that relate to the disease of interest.

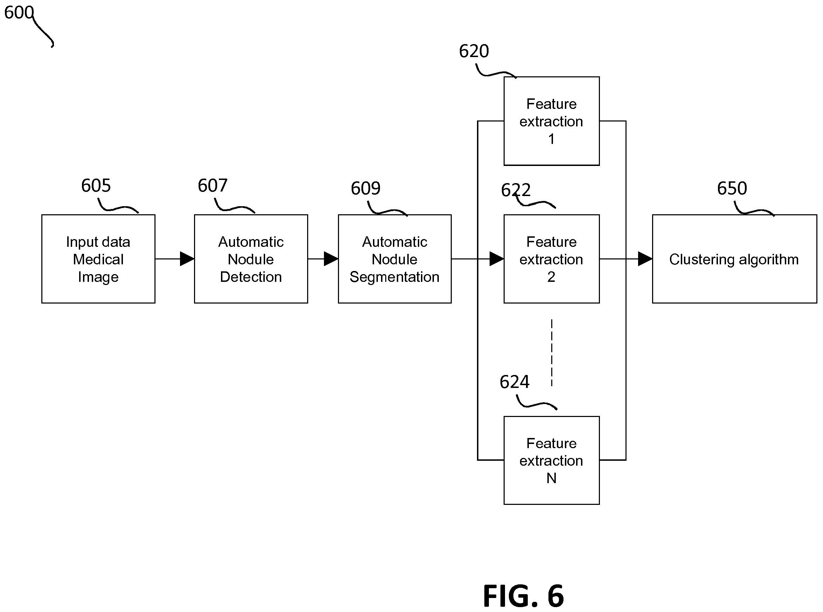

Referring now to FIG. 6 a simplified architecture 600 of an alternative example that uses automatic detection, segmentation feature extraction followed by a clustering algorithm (or performed in logic or firmware or hardware) to identify groups of nodules that have similar characteristics, is illustrated, according to examples of the present invention. The simplified architecture 600 of this embodiment is shown for lung nodules. The system extracts features within a region of interest around the nodule that has been identified through automatic nodule detection and segmentation steps.

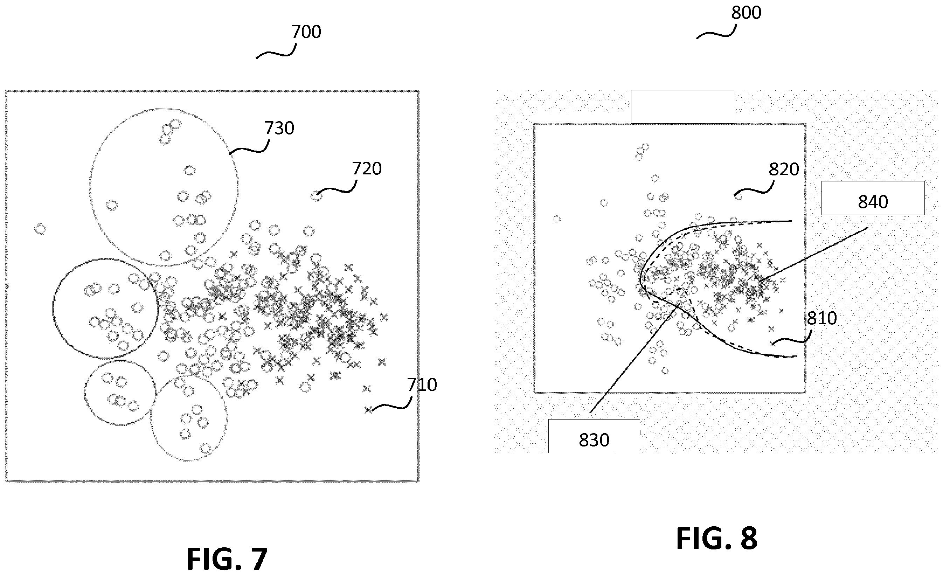

Once the features are extracted, the system then performs a cluster analysis, in order to group the related nodules together. Negative nodules within these clusters are then used to build the subset 'EN'. FIG. 7 illustrates one such example of the output of a clustering algorithm visualised in two dimensions, according to examples of the present invention. Here the crosses 710 comprise malignant nodules and the circles 720 benign nodules. The algorithm has identified four clusters of negative nodules indicated by the large circles 730. These nodules share certain characteristics, such as size, shape or appearance that should make it easier for the classifier to distinguish them from others.

### Example 5

The fifth example builds the selection of the EN set within the classifier training process in an iterative manner as described below. The system automatically analyses those cases that are easier to classify than others and assigns to the EN set automatically in each iteration until convergence or a fixed number of iterations has completed.

Referring now to FIG. 8, an example of a determination of easier and difficult negative cases in a two dimensional feature space is illustrated, according to some examples of the present invention. The solid line identifies the decision boundary of a classifier trained in the conventional manner, where all training cases are equally weighted. The dashed line shows a decision boundary that could arise once the system is trained, with the weights increased for the easier negatives. In effect, the increased weighting pulls the decision boundary towards the easier negatives. Here the feature space of the classifier is represented in two dimensions with the crosses 810 and circles 820 indicating the positive and negative training cases respectively and the curved line the decision boundary of the classifier. Recall that the aim is to classify all the crosses 810, in this example representing the malignant cases, correctly whilst maximising the number of circles 820, in this example representing the benigns, which are correctly classified.

It is clear that, at least within this two dimensional feature space, a number of the circles 820 have no hope of being discriminated from the crosses 810; those are the ones totally surrounded by a cloud of crosses 810 and are labelled as `difficult negative cases' 840 in FIG. 8. In contrast, the circles 820 that are relatively very far away from both the crosses 810 and the decision boundary of the classifier, labelled as 'easier negative cases' 830, are easy for the classifier to discriminate and therefore should be assigned to 'EN'. Yet, all the circles 820 (negative cases) are weighted equally in a standard classifier. In this example embodiment of the invention, the easy cases are automatically determined through an iterative procedure and assigned to the EN set and re-weighted for the next iteration's training step. In this embodiment, in some examples, examples of the computer-aided risk assessment may also define a set as Impossible Benign (IB) as those negative cases that are completely surrounded by positive cases and far away from the decision boundary.

In some examples, the fifth embodiment works in the following way:
1) Set the weights of all examples in the training dataset to be the same (e.g. '1').
2) Train a classifier on the above training dataset;
3) Adjust the weight of each case in the training dataset according to a method that measures the relative difficulty in classifying that case, derived from the classification performance after training.
4) Repeat steps 2 and 3 until convergence, or for a fixed number of iterations.

There are a number of ways in which to derive the measure, M, to select EN and IB from each run of classifier training:
1) Select all those negatives that are correctly classified and those negatives that are incorrectly classified but are sufficiently far away from positives (see FIG. 8). This would select all cases that the classifier correctly classifies and those that it could find "easy".
2) Alternatively, as above for (1), but calculate a distance of each of the training cases from some specific points within the dataset, for example this could be the centre of mass of the negative classes that are misclassified.
3) Alternatively, select all those negative cases that remain correctly classified across multiple random selections of training subsets. Here subsets of the training dataset are used to train the classifier multiple times and those cases that are always correctly classified are selected for EN. For this to operate correctly, sampling with replacement is necessary in building the subsets.
4) Alternatively, select as Impossible Benign (IB), all those negatives that are totally indistinguishable from the positive cases. Set the weight for IB to lower than the other cases or to zero.
5) Alternatively, a number of variations of the above approaches are also envisioned, including using the distance of the 'EN' cases as weights in the classifier or calculating the distances on a separate validation set rather than on the training set.

Thus, examples of the invention provide a system that can be trained, such that it optimises performance whilst taking into account the overall clinical context. In the example discussed, it is not necessary to correctly classify all nodules to obtain clinical and economic benefit. In contrast, the goal is to maximise the number of benign nodules that are correctly classified, whilst not misclassifying any malignant nodules.

Although examples of the invention have been described with reference to the system being used to assist in the interpretation of Chest images and Lung Nodules, it is envisaged that the concepts described herein may be employed beyond this area of the human body. For example, in other examples, it is envisaged that the concepts may be applied in any medical application where it is important to consider other aspects of the clinical context, such as economic and patient preferences.

Although examples of the invention have been described with reference to the system using a Support Vector Machine for classification, it is envisaged that the concepts described herein may be employed by any machine learning algorithm and can be for regression or classification There are many regression algorithms that may be suitable, including Random Forests, Support Vector Machines and Convolutional Neural Networks.

Although examples of the invention have been described with reference to an interpretation of medical images in a clinical setting e.g. in radiology, cardiology, oncology, it is envisaged that in other examples, the concepts described herein may be employed, say, within a clinical trial for a medical intervention e.g. drug, radiation therapy, etc.

In some examples, the medical training data being assembled may include images, blood test results, genetic profiling. In some examples, the separated sets that contain only patients with the same attribute may include benign and malignant attributes. In some examples, a data subset that includes benign nodules may be identified as those that should be further reviewed. In some examples, a data subset that includes benign nodules may be identified as those that should be discarded from further review. In some examples, medical images being assembled may include chest computed tomography (CT) scans that show at least one pulmonary nodule. In some examples, medical data related to at least one patient may include training data that further include (in addition to medical images) at least one of: meta data and historical data.

In some examples, parsing may include: first parsing the set of medical images into at least a first attribute and a second attribute based on whether or not the at least one patient has a condition; and second parsing at least one of the first attribute and second attribute into at least two data subsets, where a first attribute is estimated to be easier to classify than a second attribute. In some examples, a second weight may be associated to at least one of: the non-weighted data subsets or the non-weighted attribute. In some examples, the system may estimate one more of the aforementioned embodiments 1-5 as being easierto classify. In some examples, the condition may encompass the patient having a disease, and not just symptoms.

The present invention has been described with reference to the accompanying drawings. However, it will be appreciated that the present invention is not limited to the specific examples herein described and as illustrated in the accompanying drawings. Furthermore, because the illustrated embodiments of the present invention may for the most part, be implemented using electronic components and circuits known to those skilled in the art, details will not be explained in any greater extent than that considered necessary as illustrated above, for the understanding and appreciation of the underlying concepts of the present invention and in order not to obfuscate or distract from the teachings of the present invention.

The invention may be implemented in a computer program for running on a computer system, at least including code portions for performing steps of a method according to the invention when run on a programmable apparatus, such as a computer system or enabling a programmable apparatus to perform functions of a device or system according to the invention.

A computer program is a list of instructions such as a particular application program and/or an operating system. The computer program may for instance include one or more of: a subroutine, a function, a procedure, an object method, an object implementation, an executable application, an applet, a servlet, a source code, an object code, a shared library/dynamic load library and/or other sequence of instructions designed for execution on a computer system. Therefore, some examples describe a non-transitory computer program product having executable program code stored therein for training a CADx device, the program code operable for training a computer aided risk assessment includes: receiving and assembling training data that comprises medical data of patients that have been identified as having at least one medical condition and medical data of patients that have been identified as not having the at least one medical condition. The method further includes separating the assembled training data into data sets such that a first data set contains only the medical data of patients that have the at least one medical condition and a second data set contains only the medical data of patients that do not have the condition. The method further includes parsing at least one of the data sets into at least two subsets, whereby a first subset is distinguished over a second subset of the at least two subsets by at least one attribute. The method further includes associating different weights to the separated assembled training data, such that the first subset is prioritised during training. The output is a trained model that is used in a CADx system, for example a risk assessment system.

The computer program may be stored internally on a tangible and non-transitory computer readable storage medium or transmitted to the computer system via a computer readable transmission medium. All or some of the computer program may be provided on computer readable media permanently, removably or remotely coupled to an information processing system. The tangible and non-transitory computer readable media may include, for example and without limitation, any number of the following: magnetic storage media including disk and tape storage media; optical storage media such as compact disk media (e.g., CD-ROM, CD-R, etc.) and digital video disk storage media; non-volatile memory storage media including semiconductor-based memory units such as FLASH memory, EEPROM, EPROM, ROM; ferromagnetic digital memories; MRAM; volatile storage media including registers, buffers or caches, main memory, RAM, etc.

A computer process typically includes an executing (running) program or portion of a program, current program values and state information, and the resources used by the operating system to manage the execution of the process. An operating system (OS) is the software that manages the sharing of the resources of a computer and provides programmers with an interface used to access those resources. An operating system processes system data and user input, and responds by allocating and managing tasks and internal system resources as a service to users and programs of the system.

The computer system may for instance include at least one processing unit, associated memory and a number of input/output (I/O) devices. When executing the computer program, the computer system processes information according to the computer program and produces resultant output information via I/O devices.

In the foregoing specification, the invention has been described with reference to specific examples of embodiments of the invention. It will, however, be evident that various modifications and changes may be made therein without departing from the scope of the invention as set forth in the appended claims and that the claims are not limited to the specific examples described above.

Those skilled in the art will recognize that the boundaries between logic blocks are merely illustrative and that alternative embodiments may merge logic blocks or circuit elements or impose an alternate decomposition of functionality upon various logic blocks or circuit elements. Thus, it is to be understood that the architectures depicted herein are merely exemplary, and that in fact many other architectures can be implemented which achieve the same functionality.

Any arrangement of components to achieve the same functionality is effectively 'associated' such that the desired functionality is achieved. Hence, any two components herein combined to achieve a particular functionality can be seen as 'associated with' each other such that the desired functionality is achieved, irrespective of architectures or intermediary components. Likewise, any two components so associated can also be viewed as being 'operably connected,' or 'operably coupled,' to each other to achieve the desired functionality.

Furthermore, those skilled in the art will recognize that boundaries between the above described operations merely illustrative. The multiple operations may be combined into a single operation, a single operation may be distributed in additional operations and operations may be executed at least partially overlapping in time. Moreover, alternative embodiments may include multiple instances of a particular operation, and the order of operations may be altered in various other embodiments.

However, other modifications, variations and alternatives are also possible. The specifications and drawings are, accordingly, to be regarded in an illustrative rather than in a restrictive sense.

In the claims, any reference signs placed between parentheses shall not be construed as limiting the claim. The word 'comprising' does not exclude the presence of other elements or steps then those listed in a claim. Unless stated otherwise, terms such as 'first' and 'second' are used to arbitrarily distinguish between the elements such terms describe. Thus, these terms are not necessarily intended to indicate temporal or other prioritization of such elements.

## Claims

1. A Computer Aided Diagnosis, CAD, training system (400) for training a CAD device (310) to produce an output that indicates a probability of a patient having a medical condition of a malignant pulmonary nodule;
wherein the CAD device comprises:
an input circuit (305) configured to receive and assemble training data that comprises:
a set of positive cases (P) consisting of medical data of patients that have been identified as having at least one medical condition, wherein the at least one medical condition comprises a malignant pulmonary nodule, and
a set of negative cases (N) consisting of medical data of patients that have been identified as not having-the at least one medical condition ,
wherein each negative case is assigned a number indicating a degree of membership of a subset "Easier Negative" (EN) of cases easier to correctly classify which is the degree to which one expert considers the case easier to classify than others in the set of negative cases;
a parsing circuit (315) coupled to the input circuit (305) and configured to:
separate the assembled training data into data sets, such that a first data set contains only the medical data of patients that have the at least one medical condition and a second data set contains only the medical data of patients that do not have the at least one condition; and
parse at least one of the data sets into at least two subsets (328), whereby a first subset is distinguished over a second subset of the at least two subsets (328) by at least one clinical attribute or radiological attribute; and
a data classifier circuit (330) coupled to the parsing circuit (315) and configured to:
associate different weights to the separated assembled training data, such that the set of positive cases (P) and the subset "Easier Negative" (EN) are weighted to a greater degree than the negative cases not in the subset EN ; and
produce an output from the CAD device that indicates the probability of the patient having the at least one medical condition based on the patients at least one input medical image received and processed by the CAD device (310) with the data classifier circuit (330) trained on the separated assembled training data with the associated different weights,
wherein the data classifier circuit (330) is configured as one from a group of: Random Forest, Support Vector machine, a Convolutional Neural Network..

2. The CAD device training system (400) of any preceding Claim wherein the classifier circuit includes a feature extraction circuit (320, 322, 326) arranged to extract features of the separated assembled training data and use data analysis techniques to identify features of patient medical data that group together as being easier to classify in relation to the at least one medical condition.

3. The CAD device training system (400) of Claim 2 wherein the identified features of patient medical data that group together comprise features that are a distance greater than a feature space threshold from positive cases that require further review by a clinician.

4. The CAD device training system (400) of any preceding Claim wherein the data classifier circuit (330) is configured to update weights associated to the separated assembled training data over iterative training operations.

5. The CAD device training system (400) of any preceding Claim wherein the medical data comprises at least one from a group of: medical images, blood test results, genetic profiling, relevant to the diagnosis of pulmonary nodules.

6. A method (200) of training a Computer Aided Diagnosis, CAD, device, the method (200) comprising:
receiving and assembling training data (210) that comprises a set of positive cases (P) consisting of medical data of patients that have been identified as having at least one medical condition, wherein the at least one medical condition comprises a malignant pulmonary nodule and a set of negative cases (N) consisting of medical data of patients that have been identified as not having the at least one medical condition;
wherein each negative case is assigned a number indicating a degree of membership of a subset "Easier Negative (EN) of cases easier to correctly classify which is the degree to which one expert considers the case easier to classify than others in the set of negative cases;
separate the assembled training data into data sets, such that a first data set contains only the medical data of patients that have the at least one medical condition and a second data set contains only the medical data of patients that do not have the at least one condition; and
parsing (230) at least one of the data sets, into at least two subsets (328), whereby a first subset is distinguished over a second subset of the at least two subsets (328) by at least one clinical attribute or radiological attribute ;
associating different weights (240) to the separated assembled training data, such that the set of positive cases (P) and the subset "Easier Negative" (EN) are weighted to a greater degree than the negative cases not in the subset EN; and
producing an output from the CAD device that indicates a probability of the patient having the at least one medical condition based on the at least one medical condition a malignant pulmonary nodule based on the patients at least one input medical image received and processed by the CAD device (310) with the data classifier circuit (330) trained on the separated assembled training data with the associated different weights,
wherein the data classifier circuit (330) is configured as one from a group of: Random Forest, Support Vector machine, a Convolutional Neural Network.

7. The method (200) of Claim 6 further comprising:
extracting features of the separated assembled training data; and
identifying features of patient medical data that group together as being easier to classify in relation to the at least one medical condition.

## Patentansprüche

1. Trainingssystem (400) zur computergestützten Diagnose (CAD) zum Trainieren einer CAD-Vorrichtung (310), um eine Ausgabe zu erzeugen, die eine Wahrscheinlichkeit dafür angibt, dass ein Patient eine Erkrankung eines bösartigen Lungenknotens aufweist;
wobei die CAD-Vorrichtung Folgendes umfasst:
eine Eingangsschaltung (305), die dazu konfiguriert ist, Trainingsdaten zu empfangen und zusammenzustellen, die Folgendes umfassen:
einen Satz positiver Fälle (P), der aus medizinischen Daten von Patienten besteht, die als mindestens eine Erkrankung aufweisend identifiziert wurden, wobei die mindestens eine Erkrankung einen bösartigen Lungenknoten umfasst, und
einen Satz negativer Fälle (N), der aus medizinischen Daten von Patienten besteht, die als nicht die mindestens eine Erkrankung aufweisend identifiziert wurden,
wobei jedem negativen Fall eine Zahl zugewiesen wird, die einen Zugehörigkeitsgrad zu einem Teilsatz "Leichter negativ" (EN) von Fällen, die leichter korrekt zu klassifizieren sind, angibt, bei dem es sich um den Grad handelt, in dem ein Fachmann den Fall als leichter zu klassifizieren ansieht als andere in dem Satz negativer Fälle;
eine Parsing-Schaltung (315), die an die Eingangsschaltung (305) gekoppelt und zu Folgendem konfiguriert ist:
Trennen der zusammengestellten Trainingsdaten in Datensätze derart, dass ein erster Datensatz nur die medizinischen Daten von Patienten enthält, welche die mindestens eine Erkrankung aufweisen, und ein zweiter Datensatz nur die medizinischen Daten von Patienten enthält, welche nicht die mindestens eine Erkrankung aufweisen; und
Parsen mindestens eines der Datensätze in mindestens zwei Teilsätze (328), wodurch ein erster Teilsatz gegenüber einem zweiten Teilsatz der mindestens zwei Teilsätze (328) durch mindestens ein klinisches Attribut oder ein radiologisches Attribut unterschieden wird; und
eine Datenklassifizierungsschaltung (330), die an die Parsing-Schaltung (315) gekoppelt und zu Folgendem konfiguriert ist:
Zuordnen von unterschiedlichen Gewichtungen zu den getrennten zusammengestellten Trainingsdaten derart, dass der Satz positiver Fälle (P) und der Teilsatz "Leichter negativ" (EN) in einem höheren Grad gewichtet werden als die negativen Fälle, die sich nicht in dem Teilsatz EN befinden; und
Erzeugen einer Ausgabe aus der CAD-Vorrichtung, welche die Wahrscheinlichkeit dafür angibt, dass ein Patient die mindestens eine Erkrankung aufweist, basierend auf mindestens einem medizinischen Eingangsbild des Patienten, das durch die CAD-Vorrichtung (310) empfangen und mit der anhand der getrennten zusammengestellten Trainingsdaten mit den zugeordneten Gewichtungen trainierten Klassifizierungsschaltung (330) verarbeitet wurde,
wobei die Datenklassifizierungsschaltung (330) konfiguriert ist als eines von einer Gruppe bestehend aus: Random Forest, Support-Vector-Maschine, einem neuronalen Faltungsnetzwerk.

2. Trainingssystem (400) für CAD-Vorrichtung nach einem der vorhergehenden Ansprüche, wobei die Klassifizierungsschaltung eine Merkmalsextraktionsschaltung (320, 322, 326) beinhaltet, die dazu angeordnet ist, Merkmale der getrennten zusammengestellten Trainingsdaten zu extrahieren und Datenanalysetechniken zu verwenden, um Merkmale von medizinischen Patientendaten zu identifizieren, die zusammen eine Gruppe bilden, welche in Bezug auf die mindestens eine Erkrankung leichter zu klassifizieren ist.

3. Trainingssystem (400) für CAD-Vorrichtung nach Anspruch 2, wobei die identifizierten Merkmale der medizinischen Patientendaten, die zusammen eine Gruppe bilden, Merkmale umfassen, die um einen Abstand größer als ein Merkmalsraumschwellenwert von positiven Fällen sind, welche eine weitere Prüfung durch einen Kliniker erfordern.

4. Trainingssystem (400) für CAD-Vorrichtung nach einem der vorhergehenden Ansprüche, wobei die Datenklassifizierungsschaltung (330) dazu konfiguriert ist, Gewichtungen zu aktualisieren, die den getrennten zusammengestellten Trainingsdaten über iterative Trainingsvorgänge zugeordnet werden.

5. Trainingssystem (400) für CAD-Vorrichtung nach einem der vorhergehenden Ansprüche, wobei die medizinischen Daten mindestens eines von einer Gruppe umfassen, die aus Folgendem besteht: medizinischen Bildern, Bluttestergebnissen, Erstellung genetischer Profile, relevant für die Diagnose von Lungenknoten.

6. Verfahren (200) zum Trainieren einer Vorrichtung zur computergestützten Diagnose (CAD), wobei das Verfahren (200) Folgendes umfasst:
Empfangen und Zusammenstellen von Trainingsdaten (210), die einen Satz positiver Fälle (P), der aus medizinischen Daten von Patienten besteht, die als mindestens eine Erkrankung aufweisend identifiziert wurden, wobei die mindestens eine Erkrankung einen bösartigen Lungenknoten umfasst, und einen Satz negativer Fälle (N) umfassen, der aus medizinischen Daten von Patienten besteht, die als nicht die mindestens eine Erkrankung aufweisend identifiziert wurden;
wobei jedem negativen Fall eine Zahl zugewiesen wird, die einen Zugehörigkeitsgrad zu einem Teilsatz "Leichter negativ" (EN) von Fällen, die leichter korrekt zu klassifizieren sind, angibt, bei dem es sich um den Grad handelt, in dem ein Fachmann den Fall als leichter zu klassifizieren ansieht als andere in dem Satz negativer Fälle;
Trennen der zusammengestellten Trainingsdaten in Datensätze derart, dass ein erster Datensatz nur die medizinischen Daten von Patienten enthält, welche die mindestens eine Erkrankung aufweisen, und ein zweiter Datensatz nur die medizinischen Daten von Patienten enthält, welche nicht die mindestens eine Erkrankung aufweisen; und
Parsen (230) mindestens eines der Datensätze in mindestens zwei Teilsätze (328), wodurch ein erster Teilsatz gegenüber einem zweiten Teilsatz der mindestens zwei Teilsätze (328) durch mindestens ein klinisches Attribut oder ein radiologisches Attribut unterschieden wird;
Zuordnen von unterschiedlichen Gewichtungen (240) zu den getrennten zusammengestellten Trainingsdaten derart, dass der Satz positiver Fälle (P) und der Teilsatz "Leichter negativ" (EN) in einem höheren Grad gewichtet werden als die negativen Fälle, die sich nicht in dem Teilsatz EN befinden; und
Erzeugen einer Ausgabe aus der CAD-Vorrichtung, welche eine Wahrscheinlichkeit dafür angibt, dass der Patient die mindestens eine Erkrankung aufweist, basierend auf der mindestens einen Erkrankung eines bösartigen Lungenknotens basierend auf mindestens einem medizinischen Eingangsbild des Patienten, das durch die CAD-Vorrichtung (310) empfangen und mit der anhand der getrennten zusammengestellten Trainingsdaten mit den zugeordneten Gewichtungen trainierten Klassifizierungsschaltung (330) verarbeitet wurde,
wobei die Datenklassifizierungsschaltung (330) konfiguriert ist als eines von einer Gruppe bestehend aus: Random Forest, Support-Vector-Maschine, einem neuronalen Faltungsnetzwerk.

7. Verfahren (200) nach Anspruch 6, ferner umfassend:
Extrahieren von Merkmalen der getrennten zusammengestellten Trainingsdaten; und
Identifizieren von Merkmalen von medizinischen Patientendaten, die zusammen eine Gruppe bilden, welche in Bezug auf die mindestens eine Erkrankung leichter zu klassifizieren ist.

## Revendications

1. Système d'apprentissage (400) au diagnostic assisté par ordinateur, CAD, pour former un dispositif CAD (310) sur le fait de produire une sortie qui indique une probabilité qu'un patient présente un problème de santé lié à un nodule pulmonaire malin ;
dans lequel le dispositif CAD comprend :°
un circuit d'entrée (305) configuré pour recevoir et assembler des données d'apprentissage qui comprend : un ensemble de cas positifs (P) constitué de données médicales de patients qui ont été identifiés comme présentant au moins un problème de santé, dans lequel l'au moins un problème de santé comprend un nodule pulmonaire malin, et
un ensemble de cas négatifs (N) constitué de données médicales de patients qui ont été identifiés comme ne présentant pas l'au moins un problème de santé,
dans lequel chaque cas négatif se voit attribuer un numéro indiquant un degré d'appartenance à un sous-ensemble « Négatif plus facile »(EN) de cas plus faciles à classer correctement, qui est le degré auquel un expert considère le cas plus facile à classer que d'autres dans l'ensemble des cas négatifs°;°
un circuit d'analyse (315) couplé au circuit d'entrée (305) et configuré pour :°
séparer les données d'apprentissage assemblées en ensembles de données, de sorte qu'un premier ensemble de données ne contienne que les données médicales des patients présentant au moins une condition médicale et qu'un second ensemble de données contienne seulement les données médicales des patients qui ne présentent pas l'au moins un problème ; et
analyser au moins un des ensembles de données en au moins deux sous-ensembles (328), moyennant quoi un premier sous-ensemble est distingué d'un second sous-ensemble des au moins deux sous-ensembles (328) par au moins un attribut clinique ou un attribut radiologique ; et
un circuit classificateur de données (330) couplé au circuit d'analyse (315) et configuré pour :
associer des poids différents aux données d'apprentissage assemblées séparées, de sorte que l'ensemble des cas positifs (P) et le sous-ensemble « Négatif plus facile » (EN) soient dans une plus grande mesure pondérés que les cas négatifs ne faisant pas partie du sous-ensemble EN ; et
produire une sortie du dispositif CAD qui indique la probabilité que le patient présente l'au moins un problème de santé sur la base de l'au moins une image médicale d'entrée reçue des patients et traitée par le dispositif CAD (310) avec le circuit classificateur de données (330) formé sur les données d'apprentissage assemblées séparées avec les différentes pondérations associées,
dans lequel le circuit classificateur de données (330) est configuré comme faisant partie d'un groupe de : Forêt aléatoire, machine à vecteurs de support, un réseau de neurones convolutifs.

2. Système d'apprentissage de dispositif CAD (400) selon une quelconque revendication précédente, dans lequel le circuit classificateur comporte un circuit d'extraction de caractéristiques (320, 322, 326) agencé pour extraire des caractéristiques des données de formation assemblées séparées et utiliser des techniques d'analyse de données pour identifier des caractéristiques des données médicales du patient qui se regroupent comme étant plus faciles à classer par rapport à l'au moins un problème de santé.

3. Système d'apprentissage de dispositif CAD (400) selon la revendication 2 dans lequel les caractéristiques identifiées des données médicales du patient qui se regroupent comprennent des caractéristiques qui sont à une distance supérieure à un seuil d'espace de caractéristiques des cas positifs qui nécessitent un examen plus approfondi par un clinicien.

4. Système d'apprentissage de dispositif CAD (400) selon une quelconque revendication précédente, dans lequel le circuit classificateur de données (330) est configuré pour mettre à jour les pondérations associées aux données d'apprentissage assemblées séparées sur des opérations d'apprentissage itératives.

5. Système d'apprentissage de dispositif CAD (400) selon une quelconque revendication précédente, dans lequel les données médicales comprennent au moins un élément parmi un groupe : d'images médicales, de résultats de tests sanguins, de profilage génétique, pertinents pour le diagnostic de nodules pulmonaires.

6. Procédé (200) d'apprentissage de dispositif de diagnostic assisté par ordinateur, CAD, le procédé (200) comprenant :
la réception et l'assemblage de données d'apprentissage (210) qui comprennent un ensemble de cas positifs (P) constitué de données médicales de patients qui ont été identifiés comme présentant au moins un problème de santé, dans lequel l'au moins un problème de santé comprend un nodule pulmonaire malin et un ensemble de cas négatifs (N) constitué de données médicales de patients qui ont été identifiés comme ne présentant pas l'au moins un problème de santé ;
dans lequel chaque cas négatif se voit attribuer un numéro indiquant un degré d'appartenance à un sous-ensemble « Négatif plus facile » (EN) de cas plus faciles à classer correctement qui est le degré auquel un expert considère le cas plus facile à classer que les autres dans l'ensemble des cas négatifs ;
la séparation des données d'apprentissage assemblées en ensembles de données, de sorte qu'un premier ensemble de données ne contienne que les données médicales des patients présentant l'au moins un problème de santé et qu'un second ensemble de données contienne seulement les données médicales des patients qui ne présentent pas l'au moins un problème ; et
l'analyse(230) d'au moins un des ensembles de données, en au moins deux sous-ensembles (328), moyennant quoi un premier sous-ensemble est distingué d'un second sous-ensemble des au moins deux sous-ensembles (328) par au moins un attribut clinique ou un attribut radiologique ;
l'association de différentes pondérations (240) aux données d'apprentissage assemblées séparées, de sorte que l'ensemble de cas positifs (P) et le sous-ensemble « Négatif plus facile » (EN) soient pondérés dans une plus grande mesure que les cas négatifs ne faisant pas partie du sous-ensemble EN ; et
la production d'une sortie à partir du dispositif CAD qui indique une probabilité que le patient présente l'au moins un problème de santé, sur la base de l'au moins un problème de santé, un nodule pulmonaire malin sur la base de l'au moins une image médicale d'entrée reçue des patients et traitée par le dispositif CAD (310) avec le circuit classificateur de données (330) formé sur les données d'apprentissage assemblées séparées avec les différentes pondérations associées,
dans lequel le circuit classificateur de données (330) est configuré comme faisant partie d'un groupe : de Forêt aléatoire, de machine à vecteurs de support, d'un réseau de neurones convolutifs.

7. Procédé (200) selon la revendication 6 comprenant en outre :
l'extraction des caractéristiques des données d'apprentissage assemblées séparées ; et l'identification de caractéristiques de données médicales de patient qui se regroupent comme étant plus faciles à classer par rapport à l'au moins un problème de santé.
